# FASCICULE DE BREVET EUROPEEN

(11) **EP 0 951 474 B1**
(45) Date de publication et mention de la délivrance du brevet: **05.06.2002**
(21) Numéro de dépôt: 97952999.7
(22) Date de dépôt: 23.12.1997
(51) Int. Cl.: C07K 7/64, A61K 38/13

(54) **NOUVEAUX DERIVES DE CYCLOSPORINE, LEUR PREPARATION ET LES COMPOSITIONS PHARMACEUTIQUES QUI LES CONTIENNENT**
CYCLOSPORINDERIVATE, DEREN HERSTELLUNG UND PHARMAZEUTISCHE ZUSAMMENSETZUNGEN
NOVEL CYCLOSPORIN DERIVATIVES, METHOD OF PREPARATION AND PHARMACEUTICAL COMPOSITIONS CONTAINING THEM

(30) Priorité: 24.12.1996 FR 9615955
(43) Date de publication de la demande: 27.10.1999
(73) Titulaire: Aventis Pharma S.A., 92160 Antony (FR)
(72) Inventeur: BARRIERE, Jean-Claude, F-91400 Bures sur Yvette (FR); BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Créteil (FR); MIGNANI, Serge, F-92290 Chatenay Malabry (FR)
(86) Numéro de dépôt international: FR9702406
(87) Numéro de publication internationale: WO9828330

(56) Documents cités:
- EP-A- 0 194 972
- EP-A- 0 484 281
- WO-A-97/04005
- US-A- 4 814 323
- BILLICH A ET AL: "MODE OF ACTION OF SDZ NIM 811, A NONIMMUNOSUPPRESSIVE CYCLOSPORIN A ANALOG WITH ACTIVITY AGAINST HUMAN IMMUNODEFICIENCY VIRUS (HIV) TYPE 1: INTERFERENCE WITH HIV PROTEIN-CYCLOPHILIN A INTERACTIONS" JOURNAL OF VIROLOGY, vol. 69, no. 4, avril 1995, pages 2451-2461, XP002022423

## Description

La présente invention concerne de nouveaux dérivés de cyclosporine de formule générale 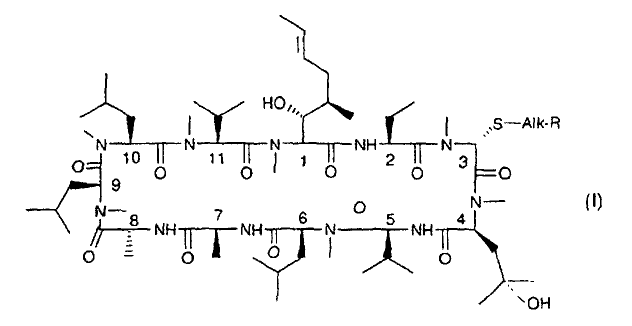 leurs sels, leur préparation et les compositions pharmaceutiques qui les contiennent.

Ces dérivés sont utiles pour le traitement et/ou la prophylaxie des infections à rétrovirus, et plus particulièrement du SIDA (syndrome d'immuno-déficience acquise) et de syndromes associés [ARC (AIDS related complex)]. Ils présentent l'avantage d'être très faiblement immunosuppresseurs.

Des dérivés de cyclosporine modifiés en position -3 ont été précédemment décrits comme agents immunosuppresseurs, dans le brevet européen EP 194972.

Des dérivés de cyclosporine diversement modifiés et notamment le dérivé 4'-hydroxy-MeLeu-4 cyclosporine, ont été précédemment décrits dans le brevet européen EP 484281 et dans Eur. J. Immunol., 17, 1359 (1987). Ces dérivés sont utiles pour le traitement du SIDA et ne sont pas immunosuppresseurs.

Dans Bioorganic and Medicinal Chemistry Letters, 6(1), 23-26 (1996) a été décrit le dérivé de cyclosporine de formule : 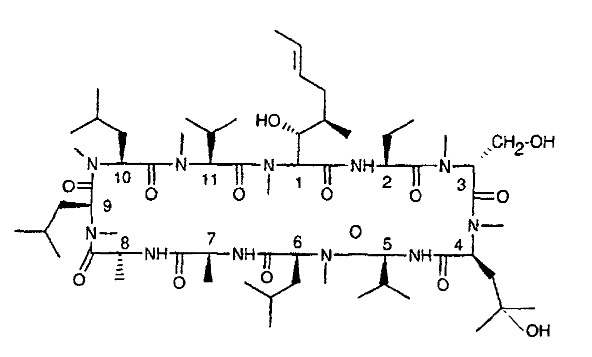 doué d'activité anti VIH-1.

Il a été trouvé maintenant que les dérivés de la cyclosporine de formule générale (I) dans laquelle :
- Alk représente un radical alcoylène contenant 2 à 6 atomes de carbone en chaîne droite ou ramifiée ou cycloalcoylène contenant 3 à 6 atomes de carbone et
- R représente
   - soit un radical hydroxy, carboxy ou alcoyloxycarbonyle,
   - soit un radical -NR₁R₂ pour lequel R₁ et R₂ identiques ou différents représentent des atomes d'hydrogène, ou des radicaux alcoyle, alcènyle (2 à 4C), cycloalcoyle (3 à 6C), phényle éventuellement substitué (par un atome d'halogène, alcoyloxy, alcoyloxycarbonyle, amino, alcoylamino ou dialcoylamino), ou représentent des radicaux benzyle ou hétérocyclyle saturé ou insaturé contenant 5 ou 6 chaînons et 1 à 3 hétéroatomes, ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle, phényle ou benzyle,
   - soit un radical de formule générale : 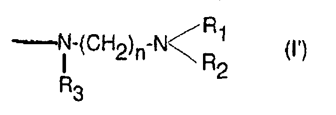 pour lequel R₁ et R₂ sont définis comme ci-dessus, R₃ représente un atome d'hydrogène ou un radical alcoyle et n est un nombre entier de 2 à 4,
les portions ou radicaux alcoyle définis ci-dessus étant droits ou ramifiés et contenant 1 à 4 atomes de carbone,
ainsi que leurs sels pharmaceutiquement acceptables lorsqu'ils existent, sont particulièrement intéressants du fait de leur activité puissante et de leur caractère très faiblement immunosuppresseur.

Dans la formule générale (I), lorsque R₁ et/ou R₂ représentent hétérocyclyle, celui-ci peut être avantageusement choisi parmi pyridyle, tétrahydropyridyle, pipéridyle, imidazolyle, oxazolyle, thiazolyle.

Lorsque R₁ et R₂ forment hétérocyclyle avec l'atome d'azote auquel ils sont attachés, à titre d'exemple, le radical hétérocyclyle peut être choisi parmi azétidinyle, pipéridyle, pipérazinyle, N-méthyl pipérazinyle, N-phényl pipérazinyle, N-benzyl pipérazinyle, pyridyle, imidazolyle, morpholino, thiomorpholino, tétrahydropyridyle, méthyl tétrahydropyridyle (par exemple 4-méthyl tétrahydropyridyle), phényl tétrahydropyridyle (par exemple 4-phényl tétrahydropyridyle).

Selon la présente invention les produits de formule générale (I) peuvent être obtenus par action d'un disulfure de formule générale :

R-Alk-S-S-Alk-R (III)

dans laquelle R et Alk sont définis comme ci-dessus, et dont le cas échéant les fonctions pouvant interférer avec la réaction ont été préalablement protégées, sur une forme activée d'un dérivé [4'-hydroxy-MeLeu]4 cyclosporine de formule : 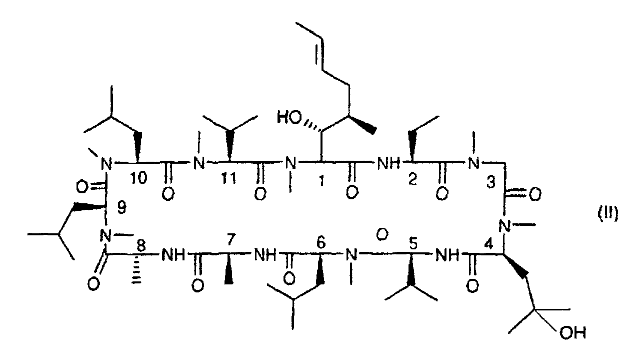 puis élimine le cas échéant le/les radicaux protecteurs.

On entend par forme activée de la cyclosporine de formule générale (II), une forme activée sur la sarcosine en position 3. Cette forme activée est préparée de préférence in situ. L'activation s'effectue généralement sous atmosphère inerte, par traitement par un dérivé organométallique (lithien notamment, comme le n-butyllithium, le diisopropyl amidure de lithium ou un mélange par exemple). Il est également possible de préparer la forme activée de la cyclosporine de formule générale (II) dans l'ammoniac liquide en présence d'un amidure alcalin (sodium, lithium par exemple), à une température comprise entre -32 et -38°C, dans un éther (notamment tétrahydrofurane, t.butyléthyléther ou un mélange).

L'addition du disulfure de formule générale (II), s'effectue avantageusement dans un solvant organique tel qu'un hydrocarbure (hexane par exemple) ou un éther (diéthyléther, tétrahydrofurane ou t.butylméthyléther par exemple) à une température comprise entre -78 et 0°C. Il est parfois préférable d'opérer sous azote.

Lorsque les substituants du radical R peuvent interférer avec la réaction, il est préférable de les protéger préalablement par des radicaux compatibles et pouvant être mis en place et éliminés sans toucher au reste de la molécule. De plus les radicaux hydroxy présents sur la cyclosporine peuvent être éventuellement protégés par tout groupement qui n'interfère pas avec la réaction.

A titre d'exemple les groupements protecteurs peuvent être choisis parmi les radicaux décrits par T.W. GREENE, Protective Groups in Organic Synthesis, J. Wiley-Interscience Publication (1991) ou par Mc Omie, Protective Groups in Organic Chemistry, Plenum Press (1973).

Le dérivé [4'-hydroxy-MeLeu]⁴ cyclosporine de formule (II) peut être préparé comme décrit dans la demande de brevet européen EP 484281.

Dans le cas où R est un radical hydroxy, il est également possible d'inverser les 2 phases de réaction, en préparant le dérivé modifié sur la sarcosine en 3 puis en effectuant l'hydroxylation. L'hydroxylation est mise en oeuvre comme mentionné précédemment pour la préparation du dérivé de formule (II).

Le disulfure de formule générale (III) peut être préparé selon ou par analogie avec les méthodes décrites dans les exemples.

Les nouveaux dérivés de la cyclosporine de formule générale (I) peuvent être purifiés le cas échéant par des méthodes physiques telles que la cristallisation ou la chromatographie.

Les dérivés de la cyclosporine selon l'invention pour lesquels R est carboxy peuvent être transformés en sels métalliques ou en sels d'addition avec une base azotée selon les méthodes connues en soi. Ces sels peuvent être obtenus par action d'une base métallique (par exemple alcaline ou alcalino terreuse), de l'ammoniaque ou d'une amine sur un produit selon l'invention, dans un solvant approprié tel que l'eau ou un alcool. Le sel formé précipite après concentration éventuelle de la solution ; il est séparé par filtration.

Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, N,N-diméthyléthanolamine, benzylamine, dicyclohexylamine, N-benzylphénéthylamine, N,N'-dibenzyléthylènediamine, diphénylènediamine, benzhydrylamine, quinine, choline, arginine, lysine, leucine, dibenzylamine).

Les dérivés de la cyclosporine selon l'invention pour lesquels R est NR₁R₂ peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, p.toluènesulfonates, iséthionates, embonates ou avec des dérivés de substitution de ces composés).

Les nouveaux dérivés de cyclosporine selon la présente invention sont particulièrement utiles pour la prophylaxie et le traitement des affections à rétrovirus et plus particulièrement du SIDA et de syndromes associés. Par prophylaxie on sous-entend notamment le traitement des sujets qui ont été exposés aux virus VIHs, en particulier les séropositifs asymptomatiques qui présentent le risque de développer la maladie dans les mois ou les années à venir après la primoinfection.

Les produits selon l'invention manifestent une activité anti-rétrovirus à des concentrations dépourvues d'effet cytotoxique ou cytostatique.

L'activité des produits de formule générale (I) a été mise en évidence dans les techniques décrites par Pauwells et Coll., J. Virol. Meth., 20, 309 (1988) et par O. Schwatz et Coll., AIDS Research and Human Retroviruses, 4(6), 441-48 (1988) et citées par J.F. Mayaux et al. Proc. Nat. Acad. Sci. USA, 91, 3564-68 (1994). Dans ces techniques les produits selon l'invention se sont montrés actifs à des concentrations de 10 à 350 nM (IC₅₀).

D'un intérêt particulier sont les produits de formule générale (I) pour lesquels :
- Alk représente un radical alcoylène contenant 2 à 6 atomes de carbone en chaîne droite ou ramifiée et
- R représente
   - soit un radical hydroxy,
   - soit un radical -NR₁R₂ pour lequel R₁ et R₂ identiques ou différents représentent des atomes d'hydrogène, ou des radicaux alcoyle, alcènyle (2 à 4C), phényle éventuellement substitué (par un atome d'halogène, alcoyloxy, alcoyloxycarbonyle, amino, alcoylamino ou dialcoylamino), ou représentent des radicaux benzyle, ou pour lequel R₁ et R₂ forment avec l'atome d'azote auquel ils sont rattachés un hétérocycle contenant 4 à 6 chainons, saturé ou insaturé, pouvant contenir un autre hétéroatome choisi parmi l'azote, l'oxygène ou le soufre et éventuellement substitué par alcoyle,
les portions ou radicaux alcoyle définis ci-dessus étant droits ou ramifiés et contenant 1 à 4 atomes de carbone, ainsi que leurs sels pharmaceutiquement acceptables lorsqu'ils existent.

Selon un aspect encore plus préférentiel de l'invention, les dérivés de cyclosporine de formule générale (I) pour lesquels :
- Alk représente un radical alcoylène contenant 2 à 5 atomes de carbone en chaîne droite ou ramifiée et
- R représente
   - soit un radical hydroxy,
   - soit un radical -NR₁R₂ pour lequel R₁ et R₂ identiques ou différents représentent des atomes d'hydrogène, ou des radicaux alcoyle, allyle, phényle ou benzyle, ou pour lequel R₁ et R₂ forment avec l'atome d'azote auquel ils sont rattachés un hétérocycle choisi parmi azétidinyle, pipéridyle, pipérazinyle, N-méthyl pipérazinyle, N-phényl pipérazinyle, N-benzyl pipérazinyle, morpholino, tétrahydropyridyle, méthyl tétrahydropyridyle, phényl tétrahydropyridyle.
les portions ou radicaux alcoyle définis ci-dessus étant droits ou ramifiés et contenant 1 à 4 atomes de carbone, ainsi que leurs sels pharmaceutiquement acceptables lorsqu'ils existent ;

Et parmi ces produits notamment les dérivés de cyclosporine ci-après :
- [(R)-2-(N,N-diméthylamino)éthylthio-Sar]³-[4'-hydroxy-MeLeu]⁴ cyclosporine A ;
- [(R)-2-(1-pipéridyl)éthylthio-Sar]³ [4'-hydroxy-MeLeu]⁴ cyclosporine A ;
- [(R)-2-(N-méthyl-N-t-butylamino)éthylthio-Sar]³ [4'-hydroxy-MeLeu]⁴ cyclosporine A ;
- [(R)-2-(hydroxy)éthylthio-Sar]³ [4'-hydroxy-MeLeu]⁴ cyclosporine A ;
- [(R)-2-(N,N-diéthylamino)éthylthio-Sar]³ [4'-hydroxy-MeLeu]⁴ cyclosporine A ;
ainsi que leurs sels pharmaceutiquement acceptables.

Les exemples suivants donnés à titre non limitatif illustrent la présente invention.

### Exemple 1 RPR130716 (essai MOC 3742 lot MOC 3743)

La [(R)-2-(N,N-diméthylamino)éthylthio-Sar]³-[4'-hydroxy-MeLeu]⁴ cyclosporine A est préparée selon la méthode suivante:

A une solution de 3,5 cm³ de diisopropylamine (préalablement distillée sur hydrure de calcium) dans 45 cm³ de tétrahydrofuranne (préalablement distillé sur sodium) refroidie à une température voisine de -10°C et sous azote, on ajoute en 10 minutes, 15,4 cm³ d'une solution de n-butyllithium 1,6 M dans l'hexane en maintenant la température à 0°C. Le mélange est agité à 0°C pendant 20 minutes, puis il est refroidi à une température voisine de -78°C. La solution ainsi obtenue est transférée, sous azote, par une canne de transfert sur une solution de 2 g de [4'-hydroxy-MeLeu]⁴-cyclosporine A dans 40 cm³ de tétrahydrofuranne préalablement refroidie à une température voisine de -76°C en maintenant la température vers -70°C. Le mélange résultant est agité à -75°C pendant 5 minutes, puis 7,2 cm³ d'une solution de n-butyllithium 1,6 M dans l'hexane sont ajoutés en 10 minutes. L'agitation est maintenue durant 10 minutes puis 6,85 g de disulfure de di-(2-N,N-diméthylaminoéthyle) sont ajoutés en 10 minutes en maintenant la température vers -75°C. Le mélange est agité à une température voisine de -78°C pendant 30 minutes puis à 0°C pendant 18 heures. On verse sur le mélange réactionnel maintenu à 0°C, 40 cm³ d'eau distillée additionnée d'acide chlorhydrique aqueux à 36 % de façon à obtenir un pH voisin de 7. Le mélange est décanté et la phase aqueuse lavée par 30 cm³ d'éther diéthylique. Les extraits organiques sont réunis, lavés avec 30 cm³ d'une solution saturée en chlorure de sodium, séchés sur sulfate de sodium, filtrés et concentrés sous pression réduite (2,7 kPa) à une température voisine de 40°C. L'huile jaune obtenue est reprise dans 40 cm³ de toluène et 300 cm³ d'eau distillée additionnée d'acide chlorhydrique à 36 % pour obtenir un pH voisin de 2. Les phases sont décantées. La phase aqueuse est relavée par 40 cm³ de toluène et les phases toluéniques rassemblées. Celles-ci sont lavées par 50 cm³ d'eau distillée acidifiée à un pH voisin de 2. Les phases aqueuses sont réunies, additionnées de 50 cm³ de toluène puis neutralisées par une solution aqueuse de bicarbonate de sodium. La phase organique est décantée, la phase aqueuse lavée deux fois par 30 cm³ de toluène. Les phases organiques sont rassemblées, séchées sur sulfate de sodium, filtrées et concentrées sous pression réduite (2,7 kPa) à une température voisine de 40°C pour donner un solide qui est purifié par chromatographie flash sur colonne de silice (0,04-0,063 mm) (éluant dichlorométhane-méthanol 98:2 en volumes puis dichlorométhane-méthanol 95:5) et en recueillant des fractions de 10 cm³. Les fractions contenant le produit attendu sont réunies et concentrées sous pression réduite (2,7 kPa) à une température voisine de 40°C pour donner un solide qui est concrété dans 3 cm³ de pentane. Après filtration, on obtient 74 mg de [(R)-2-(N,N-diméthylamino)éthylthio-Sar]³-[4'-hydroxy-MeLeu]⁴-cyclosporine A sous forme d'un solide blanc fondant vers 140°C.

Spectre de R.M.N. ¹ H (400 MHz, CDCl₃, δ en ppm) : 1,23 (d, J = 7 Hz, 3H : CH₃ 8β) ; 1,33 (d, J = 7,5 Hz, 3H : CH₃ 7β) ; 1,60 (d, J = 5 Hz, 3H : CH₃ en 1γ) ; 1,68 et 2,36 (2 dd, J = 15 et 6,5 Hz, 1H chacun : CH₂ 4β) ; 2,23 (s large, 6H : N(CH₃)₂ du 2-diméthylaminoéthylthio en 3α) ; 2,40 (mt, 1H : CH 5β) ; de 2,48 à 2,86 (mt, 4H : SCH₂CH₂N du 2-diméthylaminoéthylthio en 3α) ; 2,68 - 3,09 - 3,16 - 3,22 - 3,42 et 3,47 (6 s, respectivement 6H - 3H - 3H - 3H - 3H et 3H : 7 NCH₃) ; 3,63 (d, J = 6 Hz, 1H : OH en 1β) ; 3,72 (mt, 1H : CH 1β) ; 4,52 (mt, 1H : CH 7α) ; 4,61 (t, J = 9 Hz, 1H : CH 5α) ; 4,81 (mt, 1H : CH 8α) ; 4,95 (dd, J = 9 et 6 Hz, 1H : CH α d'une leucine) ; de 5,00 à 5,10 (mt, 2H : CH 2α et CH α d'une leucine) ; 5,10 (d, J = 11 Hz, 1H : CH 11α) ; de 5,20 à 5,35 (mt, 2H : CH=CH) ; 5,40 (t, J = 6,5 Hz, 1H : CH 4α) ; 5,47 (d, J = 6 Hz, 1H : CH 1α) ; 5,68 (dd, J = 10,5 et 4 Hz, 1H : CH α d'une leucine) ; 5,96 (s, 1H : CH en 3α) ; 7,12 (d, J = 8 Hz, 1H : CONH en 8) ; 7,46 (d, J = 9 Hz, 1H : CONH en 5) ; 7,60 (d, J = 7,5 Hz, 1H : CONH en 7) ; 7,92 (d, J = 9,5 Hz, 1H : CONH en 2).

La [4'-hydroxy-MeLeu]⁴-cyclosporine A peut être préparée comme décrit dans la demande de brevet européen EP 484281.

### Exemple 2 RPR132926 (BAM1083)

La [(R)-2-(1-pipéridyl)éthylthio-Sar]³ [4'-hydroxy-MeLeu]⁴ cyclosporine A est préparée selon la méthode suivante:

A 100 cm³ d'ammoniac maintenus à une température voisine de -33°C, on ajoute 100 mg de sodium métallique puis 100 mg de nitrate ferrique. Dès que la coloration bleue du mélange a disparu, 1,45 g de sodium métallique sont ajoutés en 20 minutes. Le mélange est agité à -33°C pendant 1 heure, une solution de 5 g de [4'-hydroxy-MeLeu]⁴ cyclosporine A dans 75 cm³ de tétrahydrofuranne est ajoutée goutte à goutte en approximativement 15 minutes puis une solution de 5,92 g de disulfure de di-[2-(1-pipéridyl)éthyle] dans 25 cm³ de dioxanne est ajoutée en 10 minutes. Le mélange réactionnel est agité à une température voisine de -33°C durant 90 minutes, puis 5 g de chlorure d'ammonium solide sont ajoutés par portions. Sous agitation, on laisse s'évaporer l'ammoniac en faisant passer la température du mélange de -33 à 25°C en 2 heures. Le mélange est filtré, le filtrat est cocentré sous pression réduite (2,7 kPa) à une température voisine de 35°C. L'huile jaune résiduelle (12,3 g) est triturée avec 50 cm³ de pentane. Le solide qui s'est formé est filtré. Le solide est trituré avec un mélange de 50 cm³ de pentane et 50 cm³ d'hexane. Le solide est filtré puis trituré à nouveau avec 25 cm³ d'heptane. Le solide est mis en solution dans 250 cm³ de t-butylméthyléther et la solution est filtrée. Le filtrat est concentré sous pression réduite (2,7 kPa) à une température voisine de 40°C pour conduire à 5,52 g d'une meringue jaune pâle. Le solide est purifié par chromatographie sur une colonne d'alumine neutre (éluant : acétate d'éthyle-cyclohexane 4:1 en volumes) et en recueillant des fractions de 10 cm³. Les fractions contenant le produit attendu sont concentrées sous pression réduite (2,7 kPa) à une température voisine de 40°C. Le solide obtenu (1,86 g) est agité 1 heure avec 25 cm³ d'acétone puis filtré et séché sous pression réduite (5 kPa) à une température voisine de 35°C. On obtient ainsi 1,72 g de [(R)-2-(1-pipéridyl)éthylthio-Sar]³ [4'-hydroxy-MeLeu]⁴ cyclosporine sous la forme d'un solide jaune fondant à une température voisine de 143°C

Spectre de R.M.N. ¹H (400 MHz, CDCl₃, δ en ppm) : 1,35 (d, J = 7,5 Hz, 3H : CH₃ 7β) ; 1,62 (d, J = 5 Hz, 3H : CH₃ 7η) ; 1,71 (dd, J = 15 et 6,5 Hz : 1H correspondant à 1H du CH₂ 4β) ; de 2,20 à 3,10 (mt : les 8H correspondant au SCH₂CH₂N et aux 2 NCH₂ de la pipéridine) ; 2,70 - 3,12 - 3,22 - 3,25 - 3,45 et 3,50 (6 s, respectivement 6H - 3H - 3H - 3H - 3H - 3H : les 7 NCH₃) ; 3,66 (d, J = 6Hz, 1H : OH en 1β) ; 3,73 (mt, 1H : CH 1β) ; 4,54 (mt, 1H : CH 7α) ; 4,62 (t large, J = 9 Hz, 1H : CH 5α) ; 4,83 (mt, 1H : CH 8α) ; 4,97 (dd, J = 8 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; de 5,25 à 5,40 (mt, 2H : CH=CH) ; 5,42 (t, J = 6,5 Hz, 1H : CH 4α) ; 5,50 (d, J = 6 Hz, 1H : CH 1α) ; 5,71 (dd, J = 10,5 et 4 Hz, 1H : CH α d'une leucine) ; 6,23 (s, 1H : CH 3α) ; 7,14 (d, J = 8 Hz, 1H : CONH en 8) ; 7,53 (d, J = 9 Hz, 1H : CONH en 5) ; 7,62 (mt, 1H : CONH en 7) ; 7,92 (d, J = 10 Hz, 1H : CONH en 2).

Le disulfure de di-[2-(1-pipéridyl)éthyle] peut être préparé selon la méthode suivante:

A une solution de 66 g de 2-(1-pipéridyl)-éthanethiol dans 850 cm³ de dichlorométhane refroidie à 0°C, on ajoute goutte à goutte en 10 minutes, 127,7 cm³ de triéthylamine puis une solution de 57,78 g d'iode dans 250 cm³ d'éther de diéthyle. Le mélange est agité durant 30 minutes, à une température voisine de 20°C puis repris par 5 g de sulfite de sodium en solution dans 500 cm³ d'eau distillée. Le pH de la phase aqueuse est ajusté à 9 par ajout d'une solution aqueuse saturée en carbonate de potassium. La phase organique est décantée, la phase aqueuse est extraite par 600 cm³ au total de dichlorométhane. Les phases organiques réunies sont séchées sur sulfate de magnésium, puis concentrées sous pression réduite (2,7 kPa) à une température voisine de 40°C pour conduire à 56,5 g de disulfure de di-[2-(1-pipéridyl)éthyle] sous la forme d'une huile marron utilisée sans autre purification.

Le 2-(1-pipéridyl)-éthanethiol peut être préparé selon la méthode suivante:

Le 2- (1-pipéridyl)-éthanethiol peut être préparé de la manière suivante: une solution de 137 g de chlorhydrate de 2-(1-pipéridyl)éthylisothiourée et de 84,3 g d'hydroxyde de sodium dans 1300 cm³ d'eau distillée est chauffée à reflux durant 90 minutes. Après avoir été ramené à une température voisine de 20°C, le mélange est neutralisé (pH = 7) par ajout d'acide chlorhydrique concentré. Le mélange est extrait par 450 cm³ au total de dichlorométhane. Les phases organiques réunies sont lavées par 100 cm³ au total d'eau distillée, séchées sur sulfate de magnésium puis concentrées sous pression réduite (2,7 kPa) à une température voisine de 40°C pour conduire à 66 g de 2-(1-pipéridyl)-éthanethiol sous la forme d'une huile utilisée sans autre purification.

Le chlorhydrate de 2-(1-pipéridyl)éthylisothiourée peut être préparé de la manière suivante:

Une suspension de 100 g de chlorhydrate de N-(2-chloroéthyl)pipéridine et de 41,5 g de thiourée dans 250 cm³ de diméthylformamide est chauffée à une température voisine de 110°C durant 2 heures. Le mélange est refroidi à une température voisine de 20°C. Le solide blanc formé est ensuite filtré, rincé avec 100 cm³ au total d'éther de diéthyle et séché sous pression réduite (10 kPa) à une température voisine de 40°C pour conduire à 134,1 g de chlorhydrate de 2-(1-pipéridyl)éthylisothiourée sous la forme d'un solide blanc fondant à une température voisine de 240°C.

### Exemple 3 RPR202946 (YOD87)

La [(R)-2-(N-méthyl-N-t-butylamino)éthylthio-Sar]³ [4'-hydroxy-MeLeu]⁴ cyclosporine A est préparée selon la méthode suivante :

A 720 cm³ d'ammoniac maintenus à une température voisine de -33°C, on ajoute 100 mg de sodium métallique puis 100 mg de nitrate ferrique. Dès que la coloration bleue du mélange a disparu, 6,50 g de sodium métallique sont ajoutés en 30 minutes. Le mélange est agité à -33°C pendant 90 minutes, une solution de 24 g de [4'-hydroxy-MeLeu]⁴ cyclosporine A dans 720 cm³ de t-butylméthyléther est ajoutée goutte à goutte en approximativement 30 minutes puis une solution de 3,5 g de disulfure de di-[2-(N-méthyl-N-t.butylamino)éthyle] dans 120 cm³ de t-butylméthyléther est ajoutée en 15 minutes. Le mélange réactionnel est agité à une température voisine de -33°C durant 30 minutes, puis 24 g de chlorure d'ammonium solide sont ajoutés par portions. Sous agitation, on laisse s'évaporer l'ammoniac en faisant passer la température du mélange de -33°C à 25°C en 12 heures. Le mélange réactionnel est filtré. Le solide est lavé avec 1800 cm³ au total de diéthyléther. Les phases organiques réunies sont concentrées sous pression réduite (2,7 kPa), à une température voisine de 40°C. L'huile résiduelle (50,3 g) est triturée durant 2 heures avec 1200 cm³ de pentane. Le solide obtenu est filtré, lavé avec 1800 cm³ d'éther de diéthyle. Le solide blanc résiduel (34,9 g) est purifié par chromatographie sur une colonne de silice (0,020-0,045 mm) (éluant : dichlorométhane-éthanol 19:1 en volumes). Les fractions contenant le produit attendu sont concentrées sous pression réduite (2,7 kPa) à une température voisine de 40°C pour donner 8,9 g d'un solide. Ce solide, trituré 12 heures avec du pentane donne après filtration et séchage à une température voisine de 40°C, 6,1 g de [(R)-2-(N-méthyl-N-t-butylamino)éthylthio-Sar]³ [4'-hydroxyMeLeu]⁴-cyclosporine A sous la forme d'un solide blanc cassé fondant vers 126-136°C.

Spectre de R.M.N. ¹H (400 MHz, CDCl₃, δ en ppm) : 1,02 (s : les 9H correspondant au C(CH₃)₃) ; 1,24 (mt : les 3H correspondant au CH₃ 8β) ; 1,33 (d, J = 7,5 Hz, 3H : CH₃ 7β) ; 1,61 (d, J = 5 Hz, 3H : CH₃ 1η) ; 1,69 et 2,36 (2 dd, J = 15 et 6,5 Hz, 1H chacun : CH₂ 4β) ; 2,18 (s large, 3H : NCH₃ du 2 N-terbutyl N-méthyl aminoéthylthio en 3α) ; de 2,50 à 2,85 (mt : les 4H correspondant au SCH₂CH₂N du 2 N-terbutyl N-méthyl aminoéthylthio en 3α) ; 2,70 - 3,11 - 3,14 - 3,24 - 3,43 et 3,48 (6 s, respectivement 6H - 3H - 3H - 3H - 3H et 3H : les 7 NCH₃) ; 3,67 (mt, 1H : OH en 1β) ; 3,72 (mt, 1H : CH 1β) ; 4,52 (mt, 1H : CH 7α) ; 4,62 (t large, J = 9 Hz, 1H : CH 5α) ; 4,82 (mt, 1H : CH 8α) ; de 4,90 à 5,10 (mt, 3H : CH α de deux leucines et CH 2α) ; 5,11 (d, J = 11 Hz, 1H : CH 11α) ; de 5,20 à 5,40 (mt, 2H : CH=CH) ; 5,41 (t, J = 6,5 Hz, 1H : CH 4α) ; 5,48 (d, J = 6 Hz, 1H : CH 1α) ; 5,68 (dd, J = 10,5 et 4 Hz, 1H : CH α d'une leucine) ; 5,87 (s, 1H : CH 3α) ; 7,14 (d, J = 8 Hz, 1H : CONH en 8) ; 7,48 (d, J = 9 Hz, 1H : CONH en 5) ; 7,64 (d, J = 8 Hz, 1H : CONH en 7) ; 7,94 (d, J = 10 Hz, 1H : CONH en 2).

Le disulfure de di-[2-(N-méthyl-N-t.butylamino)éthyle] peut être préparé de la manière suivante:

A une solution de 28,7 g de 2-(N-t-butyl-N-méthylamino)-éthanethiol dans 190 cm³ de méthanol, on ajoute 0,1 cm³ d'une solution aqueuse 1N d'hydroxyde de sodium puis on fait passer un courant d'air durant 60 heures à une température voisine de 20°C. Le méthanol est éliminé sous pression réduite (2,7 kPa). L'huile résiduelle est mise en solution dans 400 cm³ d'éther de diéthyle. La phase organique est séchée sur sulfate de magnésium, filtrée puis concentrée sous pression réduite (2,7 kPa) à une température voisine de 40°C.pour conduire à 26,6 g de disulfure de di-[2-(N-méthyl-N-t.butylamino)éthyle] sous la forme d'une huile jaune.

Le 2-(N-t.butyl-N-méthylamino)-éthanethiol peut être préparé selon la méthode suivante:

Une solution de 125 cm³ de N-t.butyl-N-méthylamine et de 50 g d'épisulfure d'éthylène dans 750 cm³ de t.butyl méthyl éther est agitée durant 48 heures à une température voisine du reflux. Le mélange est concentré sous pression réduite (10 kPa) à une température voisine de 35°C. Une distillation fractionnée sous pression réduite (5,8 kPa) du mélange réactionnel conduit à 28,7 g de 2-(N-t .butyl-N-méthylamino)-éthanethiol sous la forme d'une huile incolore bouillant entre 84 et 86°C sous 5,8 kPa.

### Exemple 4 RPR132810 (RR5923A)

La [(R)-2-(hydroxy)éthylthio-Sar]³ [4'-hydroxy-MeLeu]⁴ cyclosporine A est préparée de la manière suivante :

L'hydroxylation avec la souche Sebekia benihana est effectuée comme décrit ci-après : deux erlenmeyers de 250 cm³, contenant 50 cm³ de milieu A (peptone: 10 g; extrait de levure: 5 g; amidon: 10 g; glucose: 5 g; agar: 1 1g; eau qsp 1000 cm³; pH ajusté à 7,2; stérilisation à 121°C pendant 25 min.) sont ensemencés à 2 % à partir de deux ampoules liquides congelées de la souche *Sebekia benihana* puis sont incubés sous agitation à 220 tours/min. pendant 72 heures à une température voisine de 28°C. Ces deux erlenmeyers constituent les erlenmeyers inoculum.

Seize erlenmeyers de 250 cm³ contenant 50 cm³ de milieu B (glucose: 10 g; amidon soluble: 10 g; extrait de levure: 2,5 g; farine de soja: 12,5 g; dextrine: 10 g; dihydrogénophosphate de potassium: 0,12 g; sulfate de magnésium heptahydrate: 0,10 g; hydrogénophospate dipotassique: 0,25 g; chlorure de calcium dihydrate: 0,05 g; [ 1 cm³ d'une solution contenant: H₃BO₃ : 0,1 g; FeSO₄,7H₂O: 5 g; KI: 0,05 g; CaCl₂,6H₂O: 2 g; CuSO₄,5H₂O: 0,2g ; MnCl₂,4H₂O: 2 g; ZnSO₄,7H₂O: 4 g; (NH₄)₆Mo₇O₂₄ : 0,2 g; H₂SO₄ 97 % : 1 cm³; eau qsp: 1000 cm³]; eau qsp: 1000 cm³: pH ajusté à 7,2-7,5; stérilisation à 121°C pendant 25 min.) sont ensemencés à 4 % à partir des erlenmeyers inoculum, puis sont incubés sous agitation à 220 tours/min. pendant 40 heures à une température voisine de 28°C avant ajout du produit à biotransformé. Ils constituent les erlenmeyers de production.

Une solution de 0,192 g de [(R)-2-(hydroxy)éthylthio-Sar]³ cyclosporine A dans 8 cm³ d'éthanol est préparée extemporanément, puis filtrée sur filtre Millipore 0,2 µ. On ajoute stérilement dans chacun des erlenmeyers de production 0,5 cm³ de la solution mère de La [(R)-2-(hydroxy)éthylthio-Sar]³ cyclosporine A. Les erlenmeyers sont incubés sous agitation à 220 tours/min. à une température voisine de 28°C. Après 120 heures, chaque erlenmeyer est extrait avec 100 cm³ d'acétate d'éthyle. Les 16 phases organiques sont réunies, concentrées sous pression réduite (2,7 kPa) à une température voisine de 40°C puis reprises dans 20 cm³ de méthanol. A la solution obtenue est ajouté 5 g de silice Sorbsil C60 (40-60 µm) (Prolabo) et le tout est concentré sous pression réduite (2,7 kPa), puis séché 2 heures à l'étuve à une température voisine de 25°C. Le résidu est chromatographié sur silice Sorbsil C60 (40-60 µm) (Prolabo) éluée par un mélange d'acétate d'éthyle et de cyclohexane 4:1 (en volumes), (débit 20 cm³/heure, fraction de 6 cm³). A partir de la fraction 6 on élue avec un gradient d'acétate d'éthyle et de méthanol. Le produit attendu est élue à 6 % de méthanol. Les fractions ne contenant que le produit attendu sont rassemblées et évaporées sous pression réduite (2,7 kPa) à une température voisine de 40°C. Le résidu est repris par 2 cm³ d'acétate d'éthyle et 10 cm³ de cyclohexane puis on laisse 2 heures à une température voisine de 4°C. Le mélange est ensuite filtré, lavé à l'heptane et concentré à sec (2,7 kPa) pour donner 10 mg de [(R)-2-(hydroxy)éthylthio-Sar]³ [4'-hydroxy-MeLeu]⁴ cyclosporine A sous la forme d'une poudre blanche.

Spectre de R.M.N. ¹H (400 MHz, CDCl₃, δ en ppm) : 1,26 (d, J = 7,5 Hz, 3H : CH₃ 8β) ; 1,37 (d, J = 7,5 Hz, 3H : CH₃ 7β) ; 1,64 (d, J = 5 Hz, 3H : CH₃ 1η) ; 2,68 - 2,70 - 3,13 - 3,20 - 3,25 - 3,45 et 3,50 (7 s, 3H chacun : les 7 NCH₃) ; 2,83 (mt, 2H : SCH₂) ; 3,77 (mt, 1H : CH 1β) ; 3,82 (mt, 2H : CH₂O) ; 4,55 (mt, 1H : CH 7α) ; 4,67 (t large, J = 9 Hz, 1H : CH 5α) ; 4,85 (mt, 1H : CH 8α) ; 5,00 (dd, J = 9 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; de 5,20 à 5,35 (mt, 2H : CH=CH) ; 5,40 (t, J = 6,5 Hz, 1H : CH 4α) ; 5,49 (d, J = 6 Hz, 1H : CH 1α) ; 5,71 (dd, J = 10,5 et 4 Hz, 1H : CH α d'une leucine) ; 5,92 (s, 1H : CH 3α) ; 7,17 (d, J = 8 Hz, 1H : CONH en 8); 7,48 (d, J = 9 Hz, 1H : CONH en 5) ; 7,64 (d, J = 8 Hz, 1H : CONH en 7) ; 7,95 (d, J = 10 Hz, 1H : CONH en 2).

La [(R)-2-(hydroxy)éthylthio-Sar]³ cyclosporine A (RPR132546) peut être préparée selon la méthode suivante:

A une solution de 1,02 g de [(R)-2-(tert-butyldiméthylsilyloxy) éthylthio-Sar]³ cyclosporine A (RPR132288) dans 50 cm³ de tétrahydrofuranne on ajoute 3,7 cm³ d'une solution 1M de fluorure de tétrabutylammonium dans le tétrahydrofuranne. Le mélange est agité durant 4 heures à une température voisine de 20°C, puis 50 cm³ d'eau distillée sont ajoutés au mélange. Le tétrahydrofuranne est évaporé sous pression réduite (2,7 kPa) à une température voisine de 40°C. Le résidu est traité par 50 cm³ d'acétate d'éthyle. La phase organique est décantée et la phase aqueuse est extraite par 100 cm³ au total d'acétate d'éthyle. Les phase organiques réunies sont lavées par 100 cm³ au total d'eau distillée, séchées sur sulfate de magnésium, filtrées puis concentrées sous pression réduite (2,7 kPa) à une température voisine de 40°C. Le solide résiduel (0,88 g) est purifié par chromatographie sur une colonne de silice (0,020-0,045 mm) (éluant : acétate d'éthyle) en recueillant des fractions de 15 cm³. Les fractions contenant le produit attendu (fractions 62 à 140) sont réunies et concentrées sous pression réduite (2,7 kPa) à une température voisine de 40°C. Le solide résiduel (0,82 g) est purifié par chromatographie sur une colonne d'alumine neutre (éluant : acétate d'éthyle-cyclohexane 4:1 en volumes) en recueillant des fractions de 5 cm³. Les fractions contenant le produit attendu (fractions 18 à 288) sont réunies et concentrées sous pression réduite (2,7 kPa) à une température voisine de 40°C. Le solide résiduel (0,66 g) est traité par 50 cm3 d'acétate d'éthyle, la solution est filtrée puis évaporée sous pression réduite (2,7 kPa) à une température voisine de 40°C. Le solide résiduel est trituré avec 50 cm³ d'éther de diéthyle, puis filtré et séché sous pression réduite (3 kPa) à une température voisine de 45°C pour donner 0,65 g de [(R)-2-(hydroxy)éthylthio-Sar]³ cyclosporine A sous la forme d'un solide blanc fondant à une température voisine de 139°C.

Spectre de R.M.N. ¹H (400 MHz, CDCl₃, 8 en ppm) : 1,26 (d, J = 7,5 Hz, 3H : CH₃ 8β) ; 1,36 (d, J = 7,5 Hz, 3H : CH₃ 7β) ; 1,64 (d, J = 5 Hz, 3H : CH₃ 1η) ; 2,70 - 3,13 - 3,14 - 3,27 - 3,47 et 3,51 (6 s, respectivement 6H - 3H - 3H - 3H - 3H - 3H : les 7 NCH₃) ; 2,81 (t, J = 6 Hz, 2H : SCH₂) ; 3,78 (mt, 1H : CH 1β) ; 3,86 (mt, 2H : CH₂O) ; 4,54 (mt, 1H : CH 7α) ; 4,66 (t large, J = 9 Hz, 1H : CH 5α) ; 4,84 (mt, 1H : CH 8α) ; 4,99 (dd, J = 9 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; 5,24 (dd, J = 11 et 4 Hz, 1H : CH α d'une leucine) ; 5,33 (mt, 2H : CH=CH) ; 5,48 (d, J = 6 Hz, 1H : CH 1α) ; 5,71 (dd, J = 10,5 et 4 Hz, 1H : CH α d'une leucine) ; 5,97 (s, 1H : CH 3α) ; 7,18 (d, J = 8 Hz, 1H : CONH en 8) ; 7,30 (d, J = 9 Hz, 1H : CONH en 5) ; 7,68 (d, J = 8 Hz, 1H : CONH en 7) ; 7,98 (d, J = 10 Hz, 1H : CONH en 2).

La [(R)-2-(tert-butyldiméthylsilyloxy)éthylthio-Sar]³ cyclosporine A (RPR132288) peut être préparée selon la méthode suivante:

A 120 cm³ d'ammoniac maintenus à une température voisine de -33°C, on ajoute 100 mg de sodium métallique puis 100 mg de nitrate ferrique. Dès que la coloration bleue du mélange a disparu, 1,3 g de sodium métallique sont ajoutés en 45 minutes. Le mélange est agité à -33°C pendant 30 minutes, puis une solution de 4,8 g de cyclosporine A dans 120 cm³ de tétrahydrofuranne est ajoutée goutte à goutte en approximativement 30 minutes. Le mélange est agité 30 minutes à une température voisine de -33°C puis une solution de 30,56 g de disulfure de [2-(t.butyldiméthylsilyloxy)éthyle] dans 30 cm³ de tétrahydrofuranne est ajoutée en 20 minutes. Le mélange réactionnel est agité à une température voisine de -33°C durant 2 heures, puis 4 g de chlorure d'ammonium solide sont ajoutés par portions. Sous agitation, on laisse s'évaporer l'ammoniac en faisant passer la température du mélange de -33 à 25°C en 19 heures. Le mélange est dilué par 120 cm³ d'eau distillée puis 120 cm³ d'éther de diéthyle. La phase organique est décantée, la phase aqueuse est extraite par 240 cm³ au total d'éther de diéthyle. Les phases organiques réunies sont lavées par 240 cm³ au total d'eau distillée, séchées sur sulfate de magnésium, filtrées puis concentrées sous pression réduite (2,7 kPa) à une température voisine de 40°C. Le solide résiduel (28,47 g) est purifié par chromatographie sur une colonne de silice (0,020-0,045 mm) (éluant : acétate d'éthyle-méthanol 4:1 en volumes) et en recueillant des fractions de 100 cm³. Les fractions contenant le produit attendu (fractions 58 à 95) sont réunies et concentrées sous pression réduite (2,7 kPa) à une température voisine de 40°C pour donner 1,44 g d'une meringue. 0,2 g de cette meringue sont traités par 2 cm³ d'éther de diéthyle durant 12 heures. Après séchage sous pression réduite (3 kPa) à une température voisine de 20°C, on obtient 0,13 g de [(R)-2-(tert-butyldiméthylsilyloxy)éthylthio-Sar]³ cyclosporine A sous la forme d'un solide beige fondant à une température voisine de 125°C

Spectre de R.M.N. ¹H (400 MHz, CDCl₃, δ en ppm) : 0,07 (s, 6H : Si(CH₃)₂) ; 1,26 (d, J = 7,5 Hz, 3H : CH₃ 8β) ; 1,36 (d, J = 7,5 Hz, 3H : CH₃ 7β) ; 1,61 (d, J = 5 Hz, 3H : CH₃ 1η) ; 2,71 - 3,10 - 3,12 - 3,27 - 3,45 et 3,51 (6 s, respectivement 6H - 3H - 3H - 3H - 3H - 3H : les 7 NCH₃) ; de 2,65 à 2,80 (mt, 2H : SCH₂) ; 3,63 (d, J = 6 Hz, 1H : OH en 1β) ; 3,76 (mt, 1H : CH 1β) ; de 3,75 à 3,95 (mt, 2H : CH₂O) ; 4,53 (mt, 1H : CH 7α) ; 4,63 (t large, J = 9 Hz, 1H : CH 5α); 4,83 (mt, 1H : CH 8α) ; 4,96 (dd, J = 9 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; 5,22 (dd, J = 11,5 et 4 Hz, 1H : CH α d'une leucine) ; 5,33 (mt, 2H : CH=CH) ; 5,48 (d, J = 6 Hz, 1H : CH 1α) ; 5,70 (dd, J = 10,5 et 4 Hz, 1H : CH α d'une leucine) ; 6,00 (s, 1H : CH 3α) ; 7,16 (d, J = 8 Hz, 1H : CONH en 8) ; 7,33 (d, J = 9 Hz, 1H : CONH en 5) ; 7,66 (d, J = 8 Hz, 1H : CONH en 7) ; 7,93 (d, J = 10 Hz, 1H : CONH en 2).

Le disulfure de (2-(t.butyldiméthylsilyloxy)éthyle] peut être préparé selon la méthode suivante:

A une solution de 15 cm³ de disulfure de di 2-(hydroxy)éthyle dans 20 cm³ de diméthylformamide refroidie à une température voisine de 0°C on ajoute goutte à goutte 38,64 g d'imidazole en solution dans 20 cm³ de diméthylformamide. Le mélange est agité durant 30 minutes à 0°C puis une suspenssion de 93 g tert-butyldiméthylchlorosilane dans 200 cm³ de diméthylformamide est ajouté en maintenant la température voisine de 0°C. Le mélange est agité 30 minutes à une température voisine de 0°C puis le melange est réchauffé à une température voisine de 25°C en 12 heures. Le mélange est concentré sous pression réduite (2,7 kPa) à une température voisine de 65°C. L'huile résiduelle est reprise par 100 cm³ de dichlorométhane et 100 cm³ d'eau distillée. La phase organique est décantée et la phase aqueuse est extraite par 300 cm³ au total de de dichlorométhane. Les phases organiques sont réunies, séchées sur sulfate de magnésium, filtrées puis concentrées sous pression réduite (2,7 kPa) à une température voisine de 45°C. Après élimination des composés volatils par distillation sous pression réduite (20 kPa) jusqu'à une température voisine de 200°C, 41 g de disulfure de [2-(t.butyldiméthylsilyloxy) éthyle] sont obtenus sous la forme d'une huile jaune utilisée dans l'étape précédante sans autre purification.

### Exemple 5 RPR132980 (KMI176B)

La [(R)-2-(N,N-diéthylamino)éthylthio-Sar]³ [4'-hydroxy-MeLeu]⁴ cyclosporine A est préparée selon la méthode suivante :

Dans un réacteur contenant 0,89 g d'amidure de sodium et maintenu à une température voisine de -33°C, on condense 25 cm³ d'ammoniac puis une solution de 1,654 g de [4'-hydroxy-MeLeu]⁴ cyclosporine A dans 20 cm³ de t-butylméthyléther est ajoutée goutte à goutte et sous agitation en approximativement 10 minutes. Le mélange est agité durant 90 minutes à une température voisine de -33°C, puis 2,87 g de disulfure de di-[2-(N,N-diéthylamino)éthyle] sont ajoutés en approximativement 5 minutes. Le mélange réactionnel est agité à une température voisine de -33°C durant 20 minutes, puis 1,74 g de chlorure d'ammonium solide sont ajoutés en une portion. Après 10 minutes d'agitation, on laisse évaporer l'ammoniac en faisant passer la température du milieu de -33 à 25°C en 150 minutes. Le mélange réactionnel est filtré. Le solide est lavé avec 8,8 cm³ au total de t-butylméthyléther. Les phases organiques réunies sont concentrées à sec sous pression réduite (2,7 kPa), à une température voisine de 40°C. L'huile résiduelle est agitée durant 15 minutes avec un mélange de 8 cm³ de n-hexane et 80 cm³ d'heptane, Le solide obtenu est filtré puis rincé avec 15 cm³ au total de n-heptane. Le solide résiduel est mis en solution dans 25 cm³ de t-butylméthylèther puis 35 cm³ d'eau distillée sont ajoutés à la solution obtenue et le pH de la phase aqueuse est ajusté à 2 par ajout d'acide méthanesulfonique. La phase organique est décantée, la phase aqueuse est extraite par 30 cm³ au total de t-butylméthyléther. Les phases aqueuses réunies sont amenées à pH = 9 par ajout d'ammoniaque à 20 %. La phase aqueuse est alors extraite par 25 cm³ au total de t-butylméthyléther. Les phases organiques réunies sont séchées sur sulfate de sodium, filtrées, puis conentrées sous pression réduite (2,7 kPa) à une température voisine de 40°C. L'huile marron résiduelle (1,5 g) est purifiée par chromatographie sur une colonne d'alumine neutre éluée par un mélange d'acétate d'éthyle et d'éthanol 9:1 (en volumes). Les fractions contenant le produit attendu sont concentrées sous pression réduite (2,7 kPa) à une température voisine de 40°C pour donner 0,8 g d'un solide. Une aliquote de ce solide (0,077 g) est purifiée par chromatographie sur couche mince de silice préparative (éluant :acétonitrile-méthanol-ammoniac à 28% - 260:40:3 (en volumes). La silice contenant le produit attendu est prélevée et agitée avec 5 cm³ de dichlorométhane. Après filtration et évaporation sous pression réduite (2,7 kPa) à une température voisine de 40°C de la phase organique, on obtient 0,039 mg de [(R)-2-(N,N-diéthylamino)éthylthio-Sar]³ [4'-hydroxy-MeLeu]⁴-cyclosporine A sous la forme d'un solide amorphe blanc cassé fondant vers 140°C.

Spectre de R.M.N. ¹H (400 MHz, CDCl₃, δ en ppm) : 1,34 (d, J = 7,5 Hz, 3H : CH₃ 7β) ; 1,60 (d, J = 5 Hz, 3H : CH₃ 1η) ; 1,68 et 2,36 (2 dd, J = 15 et 6,5 Hz, 1H chacun : CH₂ 4β) ; de 2,45 à 2,85 (mt : les 4H correspondant au SCH₂CH₂N du 2-diéthyl aminoéthylthio en 3α) ; 2,51 (mt : les 4H correspondant au 2 NCH₂ du 2-diéthyl aminoéthylthio en 3α) ; 2,70 - 3,11 - 3,18 - 3,24 - 3,44 et 3,49 (6 s, respectivement 6H - 3H - 3H - 3H - 3H et 3H : 7 NCH₃) ; 3,63 (d, J = 6 Hz, 1H : OH en 1β) ; 3,74 (mt, 1H : CH 1β) ; 4,52 (mt, 1H : CH 7α); 4,61 (t, J = 9 Hz, 1H : CH 5α) ; 4,81 (mt, 1H : CH 8α) ; 4,97 (dd, J = 9 et 7 Hz, 1H : CH α d'une leucine) ; de 5,00 à 5,10 (mt, 2H : CH 2α et CH α d'une leucine) ; 5,12 (d, J = 11 Hz, 1H : CH 11α) ; de 5,25 à 5,40 (mt, 2H : CH=CH) ; 5,40 (t, J = 6,5 Hz, 1H : CH 4α) ; 5,47 (d, J = 6 Hz, 1H : CH 1α) ; 5,68 (dd, J = 10,5 et 4 Hz, 1H : CH α d'une leucine) ; 5,96 (s, 1H : CH 3α) ; 7,13 (d, J = 8 Hz, 1H : CONH en 8) ; 7,47 (d, J = 9 Hz, 1H : CONH en 5) ; 7,61 (d, J = 7,5 Hz, 1H : CONH en 7) ; 7,89 (d, J = 9,5 Hz, 1H : CONH en 2).

Le disulfure de di-[2-(N,N-diéthylamino)éthyle] peut être préparé selon Bretschneider et al., Montatsh. Chem., 81, 385-396 (1950).

### Exemple 6

En opérant de manière analogue à la méthode décrite dans l'exemple 1, on prépare les produits suivants :
[(R)-2-aminoéthylthio-Sar]³-[4'-hydroxy-MeLeu]⁴-cyclosporine A ;
[(R)-2-(N-méthylamino)éthylthio-Sar]³-[4'-hydroxy-MeLeu]⁴-cyclosporine A ;
[(R)-2-(N-ethylamino)éthylthio-Sar]³-[4'-hydroxy-MeLeu]⁴-cyclosporine A ;
[(R)-2-(N-iso-propylamino)éthylthio-Sar]³-[4'-hydroxy-MeLeu]⁴-cyclosporine A ;
[(R)-2-(N-tert-butylamino)éthylthio-Sar]³-[4'-hydroxy-MeLeu]⁴-cyclosporine A ;
[(R)-2-(N-phénylamino)éthylthio-Sar]³-[4'-hydroxy-MeLeu]⁴-cyclosporine A ;
[(R)-2-(N-benzylamino)éthylthio-Sar]³-[4'-hydroxy-MeLeu]⁴-cyclosporine A ;
[(R)-2-(N-méthyl-N-éthylamino)éthylthio-Sar]³-[4'-hydroxy-MeLeu]⁴-cyclosporine A ;
[(R)-2-(N-méthyl-N-iso-propylamino)éthylthio-Sar]³-[4'-hydroxy-MeLeu]⁴-cyclosporine A ;
[(R)-2-(N-méthyl-N-tert-butylamino)éthylthio-Sar]³-[4'-hydroxy-MeLeu]⁴-cyclosporine A ;
[(R)-2-(N-méthyl-N-allylamino)éthylthio-Sar]³-[4'-hydroxy-MeLeu]⁴-cyclosporine A ;
[(R)-2-(N-méthyl-N-phénylamino)éthylthio-Sar]³-[4'-hydroxy-MeLeu]⁴-cyclosporine A ;
[(R)-2-(N-méthyl-N-benzylamino)éthylthio-Sar]³-[4'-hydroxy-MeLeu]⁴-cyclosporine A ;
[(R)-2-(N,N-diéthylamino)éthylthio-Sar]³-[4'-hydroxy-MeLeu]⁴-cyclosporine A ;
[(R)-2-(N,N-di-iso-propylamino)éthylthio-Sar]³-[4'-hydroxy-MeLeu]⁴-cyclosporine A ;
[(R)-2-(N,N-diallylamino)éthylthio-Sar]³-[4'-hydroxy-MeLeu]⁴-cyclosporine A ;
[(R)-2-(1-pipéridyl)éthylthio-Sar]³-[4'-hydroxy-MeLeu]⁴-cyclosporine A ;
[(R)-3-aminopropylthio-Sar]³-[4'-hydroxy-MeLeu]⁴-cyclosporine A ;
[(R)-3-(N-méthylamino)propylthio-Sar]³-[4'-hydroxy-MeLeu]⁴-cyclosporine A ;
[(R)-3-(N-ethylamino)propylthio-Sar]³-[4' -hydroxy-MeLeu]⁴-cyclosporine A ;
[(R)-3-(N-iso-propylamino)propylthio-Sar]³-[4'-hydroxy-MeLeu]⁴-cyclosporine A ;
[(R)-3-(N-tert-butylamino)propylthio-Sar]³-[4'-hydroxy-MeLeu]⁴-cyclosporine A ;
[(R)-3-(N-phénylamino)propylthio-Sar]³-[4'-hydroxy-MeLeu]⁴-cyclosporine A ;
[(R)-3-(N-benzylamino)propylthio-Sar]³-[4'-hydroxy-MeLeu]⁴-cyclosporine A ;
[(R)-3-(N-méthyl-N-éthylamino)propylthio-Sar]³-[4'-hydroxy-MeLeu]⁴-cyclosporine A ;
[(R)-3-(N-méthyl-N-iso-propylamino)propylthio-Sar]³-[4'-hydroxy-MeLeu]⁴-cyclosporine A ;
[(R)-3-(N-méthyl-N-tert-butylamino)propylthio-Sar]³-[4'-hydroxy-MeLeu]⁴-cyclosporine A ;
[(R)-3-(N-méthyl-N-allylamino)propylthio-Sar]³-[4'-hydroxy-MeLeu]⁴-cyclosporine A ;
[(R)-3-(N-méthyl-N-phénylamino)propylthio-Sar]³-[4'-hydroxy-MeLeu]⁴-cyclosporine A ;
[(R)-3-(N-méthyl-N-benzylamino)propylthio-Sar]³-[4'-hydroxy-MeLeu]⁴-cyclosporine A ;
[(R)-2-(N,N-diméthylamino)éthylthio-Sar]³-[4'-hydroxy-MeLeu]⁴-cyclosporine A ;
[(R)-3-(N,N-diéthylamino)propylthio-Sar]³-[4'-hydroxy-MeLeu]⁴-cyclosporine A ;
[(R)-3-(N,N-di-iso-propylamino)propylthio-Sar]³-[4'-hydroxy-MeLeu]⁴-cyclosporine A ;
[(R)-3-(N,N-diallyalamino)propylthio-Sar]³-[4'-hydroxy-MeLeu]⁴-cyclosporine A ;
[(R)-3-(1-pipéridyl)propylthio-Sar]³-[4'-hydroxy-MeLeu]⁴-cyclosporine A ;
[(R)-4-aminobutylthio-Sar]³-[4'-hydroxy-MeLeu]⁴-cyclosporine A ;
[(R)-4-(N-méthylamino)butylthio-Sar]³-[4'-hydroxy-MeLeu]⁴-cyclosporine A ;
[(R)-4-(N-éthylamino)butylthio-Sar]³-[4'-hydroxy-MeLeu]⁴-cyclosporine A ;
[(R)-4-(N-iso-propylamino)butylthio-Sar]³-[4'-hydroxy-MeLeu]⁴-cyclosporine A ;
[(R)-4-(N-tert-butylamino)butylthio-Sar]³-[4'-hydroxy-MeLeu]⁴-cyclosporine A ;
[(R)-4-(N-phénylamino)butylthio-Sar]³-[4'-hydroxy-MeLeu]⁴-cyclosporine A ;
[(R)-4-(N-benzylamino)butylthio-Sar]³-[4'-hydroxy-MeLeu]⁴-cyclosporine A ;
[(R)-4-(N-méthyl-N-éthylamino)butylthio-Sar]³-[4'-hydroxy-MeLeu]⁴-cyclosporine A ;
[(R)-4-(N-méthyl-N-iso-propylamino)butylthio-Sar]³-[4'-hydroxy-MeLeu]⁴-cyclosporine A ;
[(R)-4-(N-méthyl-N-tert-butylamino)butylthio-Sar]³-[4'-hydroxy-MeLeu]⁴-cyclosporine A ;
[(R)-4-(N-méthyl-N-allylamino)butylthio-Sar]³-[4'-hydroxy-MeLeu]⁴-cyclosporine A ;
[(R)-4-(N-méthyl-N-phénylamino)butylthio-Sar]³-[4'-hydroxy-MeLeu]⁴-cyclosporine A ;
[(R)-4-(N-méthyl-N-benzylamino)butylthio-Sar]³-[4'-hydroxy-MeLeu]⁴-cyclosporine A ;
[(R)-4-(N,N-diméthylamino)butylthio-Sar]³-[4'-hydroxy-MeLeu]⁴, cyclosporine A ;
[(R)-4-(N,N-diéthylamino)butylthio-Sar]³-[4'-hydroxy-MeLeu]⁴-cyclosporine A ;
[(R)-4-(N,N-di-iso-propylamino)butylthio-Sar]³-[4'-hydroxy-MeLeu]⁴-cyclosporine A ;
[(R)-4-(N,N-diallyalamino)butylthio-Sar]³-[4'-hydroxy-MeLeu]⁴-cyclosporine A ;
[(R)-4-(1-pipéridyl)butylthio-Sar]³-[4'-hydroxy-MeLeu]⁴-cyclosporine A ;
[(R)-2-amino-2-méthylpropylthio-Sar]³-[4'-hydroxy-MeLeu]⁴-cyclosporine A ;
[(R)-2-(N,N-diméthylamino)-2-méthylpropylthio-Sar]³- [4'-hydroxy-MeLeu]⁴-cyclosporine A ;
[(R)-2-(N,N-diéthylamino)-2-méthylpropylthio-Sar]³-[4'-hydroxy-MeLeu]⁴-cyclosporine A ;
[(R)-2-(1-pipéridyl)-2-méthylpropylthio-Sar]³-[4'-hydroxy-MeLeu]⁴-cyclosporine A ;
[(R)-3-amino-3-méthylbutylthio-Sar]³-[4'-hydroxy-MeLeu]⁴-cyclosporine A ;
[(R)-3-(N,N-diméthylamino)-3-méthylbutylthio-Sar]³-[4'-hydroxy-MeLeu]⁴-cyclosporine A ;
[(R)-3-(N,N-diéthylamino)-3-méthylbutylthio-Sar]³-[4'-hydroxy-MeLeu]⁴-cyclosporine A ;
[(R)-3-(1-pipéridyl)-3-méthylbutylthio-Sar]³-[4'-hydroxy-MeLeu]⁴-cyclosporine A
[(R)-2-(1-morpholino)éthylthio-Sar]³-[4'-hydroxy-MeLeu]⁴-cyclosporine A ;
[(R)-2-(1-azétidino)éthylthio-Sar]³-[4'-hydroxy-MeLeu]⁴-cyclosporine A ;
{(R)-2-[1-(4-méthylpipérazino)]éthylthio-Sar}³-[4'-hydroxy-MeLeu]⁴-cyclosporine A ;
{(R)-2-[1-(4-phénylpipérazino)]éthylthio-Sar}³-[4'-hydroxy-MeLeu]⁴-cyclosporine A ;
{(R)-2-[1-(4-benzylpipérazino)]éthylthio-Sar}³-[4'-hydroxy-MeLeu]⁴-cyclosporine A ;
{(R)-2-[1-(4-méthyl-1,2,3,6-tétrahydropyridyl)]éthylthio-Sar}³-[4'-hydroxy-MeLeu]⁴-cyclosporine A ;
{(R)-2-[1-(4-phényl-1,2,3,6-tétrahydropyridyl)]éthylthio-Sar}³-[4'-hydroxy-MeLeu]⁴-cyclosporine A ;
[(R)-3-(1-morpholino)propylthio-Sar]³-[4'-hydroxy-MeLeu]⁴-cyclosporine A ;
[(R)-3-(1-azétidino)propylthio-Sar]³-[4'-hydroxy-MeLeu]⁴-cyclosporine A ;
{(R)-3-[1-(4-méthylpipérazino)]propylthio-Sar}³-[4'-hydroxy-MeLeu]⁴-cyclosporine A ;
{(R)-3-[1-(4-phénylpipérazino)]propylthio-Sar}³-[4'-hydroxy-MeLeu]⁴-cyclosporine A ;
{(R)-3-[1-(4-benzylpipérazino)]propylthio-Sar}³-[4'-hydroxy-MeLeu]⁴-cyclosporine A ;
{(R)-3-[1-(4-méthyl-1,2,3,6-tétrahydropyridyl)]propylthio-Sar}³-[4'-hydroxy-MeLeu]⁴-cyclosporine A ;
{(R)-3-[1-(4-méthyl-1,2,3,6-tétrahydropyridyl)]propylthio-Sar}³-[4'-hydroxy-MeLeu]⁴-cyclosporine A.

La présente invention concerne également les compositions pharmaceutiques contenant au moins un produit de formule générale (I) le cas échéant sous forme de sel, à l'état pur ou sous forme d'une association avec un ou plusieurs diluants ou adjuvants compatibles et pharmaceutiquement acceptables, ou avec un autre agent antirétrovirus, éventuellement destiné au traitement du SIDA, un agent antiviral, immunomodulateur ou antimicrobien.

La composition selon l'invention est capable de maintenir en vie les cellules infectées par un rétrovirus comme par exemple le VIH et donc de réduire la progression vers le SIDA ou de diminuer sa gravité chez les sujets déjà infectés en réduisant la mortalité des cellules infectées. Les compositions peuvent être utilisées par voie orale, parentérale, rectale ou en aérosols.

Les compositions pharmaceutiques peuvent être utilisées à titre curatif ou à titre préventif chez des sujets présentant une immunodéficience et/ou infectés par un rétrovirus. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon.

Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout aucre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, le médecin déterminera la posologie qu'il estime la plus appropriée en fonction d'un traitement préventif ou curatif, en fonction de l'âge, du poids, du degré de l'infection et des autres facteurs propres au sujet à traiter. Généralement, les doses sont comprises entre 5 et 30 mg/kg par voie orale pour un adulte.

Il a de plus été montré que les dérivés de cyclosporine de formule générale (I) manifestent un effet de synergie lorsqu'ils sont associés à d'autres agents anti-viraux actifs sur les rétrovirus. La présente invention concerne également les associations synergisantes qui contiennent au moins un dérivé de la cyclosporine de formule générale (I) et/ou le cas échéant leurs sels, et un principe actif connu pour son activité sur les rétrovirus.

Les agents connus pour leur activité sur les rétrovirus qui peuvent être associés, sont choisis parmi des agents compatibles et inertes vis à vis du dérivé de cyclosporine de formule générale (I), aussi bien dans la catégorie des traitements pharmacologiques que dans la catégorie des traitements alternatifs tels que la thérapie génique et cellulaire ou antisens. A titre non limitatif ces agents constituant les differentes classes thérapeutiques sont choisis par exemple parmi des inhibiteurs nucléosidiques (NRTI) et non nucléosidiques (NNRTI) de la reverse transcriptase [zidovudine (AZT), didanosine (DDI), didéoxycytidine (DDC), d4T, ribavirine, 3TC, névirapine ...], parmi des inhibiteurs de la protéase [comme par exemple le Saquinavir, le Ritonavir, l'Indinavir et le Nelfinavir], des inhibiteurs de l'intégrase [comme le AR177], parmi les inhibiteurs de gene thérapie ciblant les protéines régulatrices de la réplication des VIHs tels que les inhibiteurs de la protéine rev [comme par exemple le Rev M10], ou les inhibiteurs de la nucléocapside [comme par exemple les DIBAs], parmi les inhibiteurs ciblant les transcripts RNA messager specifiques de tous les VIHs comme par exemple les anti-sens [comme GEM92, GPI-2A...], parmi les inhibiteurs de la famille des modulateurs de dNTP cellulaire [comme hydroxyurée], parmi les inhibiteurs de cytokines [comme TNF], parmi les inhibiteurs d'entrée des VIHs [comme T20, SPC-3...], ainsi que parmi les agents constituants les classes therapeutiques utilisées dans les approches vaccinales tant par biotechnologie [comme HIVAC-1e, ALVAC...] que par composés agissant sur la réponse immune [comme RG-8394].

Notamment le dérivé de cyclosporine de l'exemple 1 manifeste un effet de synergie particulièrement intéressant lorsqu'il est associé avec l'AZT, le ddI, le Sasquinavir, la Nevirapine, le Ritonavir et la ribavirine et un effet additif également intéressant associé avec l'Indinavir.

Les compositions pharmaceutiques comprenant de telles associations, éventuellement en présence d'excipients pharmaceutiquement acceptables, entrent également dans le cadre de la présente invention.

L'exemple suivant illustre une composition selon l'invention.

### Exemple

On prépare une formulation administrable par voie orale et ayant la composition suivante :

| | |
|---|---|
| [(R)-2-(N,N-diméthylamino)éthylthio-Sar]³-[4'-hydroxy-MeLeu]⁴-cyclosporine | 250 mg |
| Stéarate de magnésium | 3 mg |
| Acidsol | 15 mg |
| Silice colloïdale | 2 mg |
| Lactose | 130 mg |

## Revendications

1. Un dérivé de la cyclosporine **caractérisé en ce qu'**il répond à la formule générale : 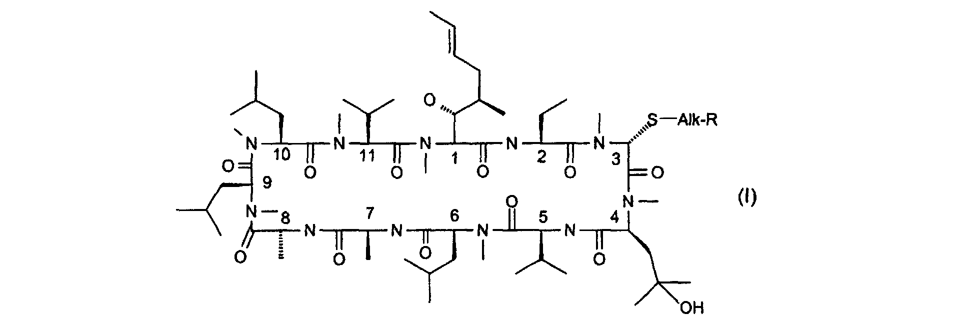 dans laquelle :
- Alk représente un radical alcoylène contenant 2 à 6 atomes de carbone en chaîne droite ou ramifiée ou cycloalcoylène contenant 3 à 6 atomes de carbone et
- R représente
• soit un radical hydroxy, carboxy ou alcoyloxycarbonyle,
• soit un radical -NR₁R₂ pour lequel R₁ et R₂ identiques ou différents représentent des atomes d'hydrogène, ou des radicaux alcoyle, alcènyle (2 à 4C), cycloalcoyle (3 à 6C), phényle éventuellement substitué (par un atome d'halogène, alcoyloxy, alcoyloxycarbonyle, amino, alcoylamino ou dialcoylamino), ou représentent des radicaux benzyle ou hétérocyclyle saturé ou insaturé contenant 5 ou 6 chaînons et 1 à 3 hétéroatomes, ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle, phényle ou benzyle,
• soit un radical de formule générale : 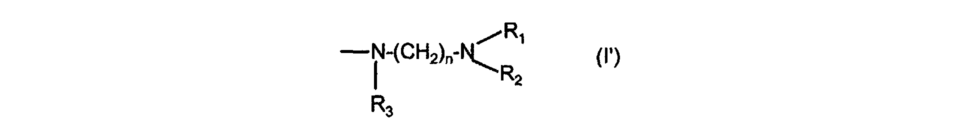 pour lequel R₁ et R₂ sont définis comme ci-dessus, R₃ représente un atome d'hydrogène ou un radical alcoyle et n est un nombre entier de 2 à 4,
les portions ou radicaux alcoyle définis ci-dessus étant droits ou ramifiés et contenant 1 à 4 atomes de carbone,
ainsi que ses sels pharmaceutiquement acceptables lorsqu'ils existent.

2. Un dérivé de la cyclosporine selon la revendication 1, **caractérisé en ce que** :
- Alk représente un radical alcoylène contenant 2 à 6 atomes de carbone en chaîne droite ou ramifiée et
- R représente
• soit un radical hydroxy,
• soit un radical -NR₁R₂ pour lequel R₁ et R₂ identiques ou différents représentent des atomes d'hydrogène, ou des radicaux alcoyle, alcènyle (2 à 4C), phényle éventuellement substitué (par un atome d'halogène, alcoyloxy, alcoyloxycarbonyle, amino, alcoylamino ou dialcoylamino), ou représentent des radicaux benzyle, ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle,
les portions ou radicaux alcoyle définis ci-dessus étant droits ou ramifiés et contenant 1 à 4 atomes de carbone, ainsi que ses sels pharmaceutiquement acceptables lorsqu'ils existent.

3. Un dérivé de la cyclosporine selon la revendication 1, **caractérisé en ce que** :
- Alk représente un radical alcoylène contenant 2 à 5 atomes de carbone en chaîne droite ou ramifiée et
- R représente
• soit un radical hydroxy,
• soit un radical -NR₁R₂ pour lequel R₁ et R₂ identiques ou différents représentent des atomes d'hydrogène, ou des radicaux alcoyle, allyle, phényle ou benzyle, ou pour lequel R₁ et R₂ forment avec l'atome d'azote auquel ils sont rattachés un hétérocycle choisi parmi azétidinyle, pipéridyle, pipérazinyle, N-méthyl pipérazinyle, N-phényl pipérazinyle, N-benzyl pipérazinyle, morpholino, tétrahydropyridyle, méthyl tétrahydropyridyle, phényl tétrahydropyridyle.
les portions ou radicaux alcoyle définis ci-dessus étant droits ou ramifiés et contenant 1 à 4 atomes de carbone, ainsi que ses sels pharmaceutiquement acceptables lorsqu'ils existent.

4. Un dérivé de la cyclosporine selon la revendication 1, **caractérisé en ce qu'**il s'agit de la [(R)-2-(N,N-diméthylamino) éthylthio-Sar]³-[4'-hydroxy-MeLeu]⁴-cyclosporine A, ainsi que ses sels pharmaceutiquement acceptables.

5. Un dérivé de la cyclosporine selon la revendication 1, **caractérisé en ce qu'**il s'agit de la [(R)-2-(1-pipéridyl)éthylthio-Sar]³ [4'-hydroxy-MeLeu]⁴ cyclosporine A, ainsi que ses sels pharmaceutiquement acceptables.

6. Un dérivé de la cyclosporine selon la revendication 1, **caractérisé en ce qu'**il s'agit de la [(R)-2-(N-méthyl-N-t-butylamino)éthylthio-Sar]³ [4'-hydroxy-MeLeu]⁴ cyclosporine A, ainsi que ses sels pharmaceutiquement acceptables.

7. Un dérivé de la cyclosporine selon la revendication 1, **caractérisé en ce qu'**il s'agit de la [(R)-2-(hydroxy)éthylthio-Sar]³ [4'-hydroxy-MeLeu]⁴ cyclosporine A, ainsi que ses sels pharmaceutiquement acceptables.

8. Un dérivé de la cyclosporine selon la revendication 1, **caractérisé en ce qu'**il s'agit de la [(R)-2-(N,N-diéthylamino)éthylthio-Sar]³ [4'-hydroxy-MeLeu]⁴ cyclosporine A, ainsi que ses sels pharmaceutiquement acceptables.

9. Procédé de préparation d'un dérivé de cyclosporine selon la revendication 1, **caractérisé en ce que** l'on fait agir un disulfure de formule générale :
R-Alk-S-S-Alk-R
dans laquelle R et Alk sont définis comme ci-dessus, et dont le cas échéant les fonctions pouvant interférer avec la réaction ont été préalablement protégées, sur une forme activée d'un dérivé [4'-hydroxy-MeLeu]⁴ cyclosporine de formule : 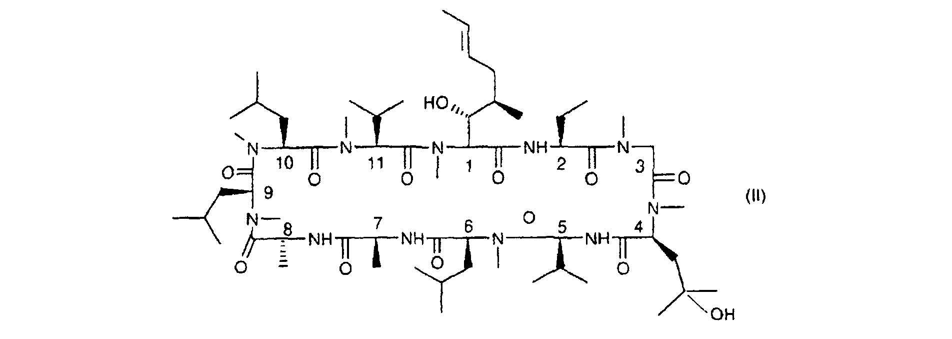 puis élimine le cas échéant les radicaux protecteurs et/ou transforme éventuellement le produit obtenu en un sel lorsqu'ils existent.

10. Composition pharmaceutique **caractérisée en ce qu'**elle contient au moins un produit selon la revendication 1, à l'état pur ou en association avec tout diluant ou adjuvant compatible et pharmaceutiquement acceptable et/ou en association avec un autre principe actif antiviral, immunomodulateur ou antimicrobien.

11. Associations synergisantes **caractérisées en ce qu'**elles comprennent au moins un dérivé de cyclosporine selon la revendication 1 et au moins un autre agent connu pour son activité anti-rétrovirus.

## Claims

1. Cyclosporin derivative, **characterized in that** it corresponds to the general formula: 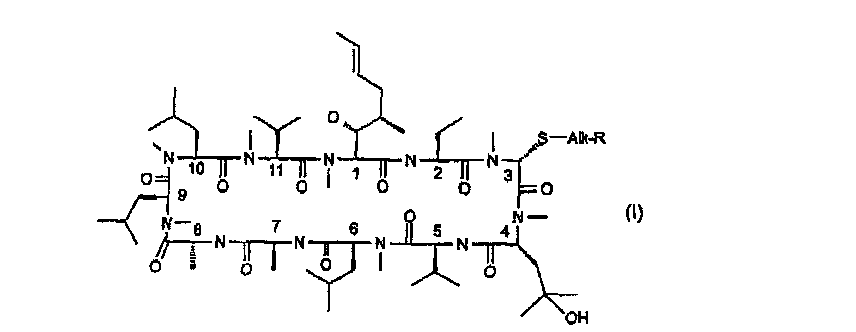 in which:
- Alk represents an alkylene radical containing 2 to 6 carbon atoms in a straight or branched chain or a cycloalkylene radical containing 3 to 6 carbon atoms and
- R represents
• either a hydroxyl, carboxyl or alkyloxycarbonyl radical,
• or an -NR₁R₂ radical in which R₁ and R₂, which are identical or different, represent hydrogen atoms or alkyl, alkenyl (2 to 4C), cycloalkyl (3 to 6C) or optionally substituted (by a halogen atom, alkyloxy, alkyloxycarbonyl, amino, alkylamino or dialkylamino) phenyl radicals or represent benzyl or heterocyclyl radicals, the heterocyclyl radical being saturated or unsaturated and containing 5 or 6 ring members and 1 to 3 heteroatoms, or in which R₁ and R₂ form, with the nitrogen atom to which they are attached, a saturated or unsaturated heterocycle containing 4 to 6 ring members, which can contain another heteroatom chosen from nitrogen, oxygen or sulphur, optionally substituted by alkyl, phenyl or benzyl,
• or a radical of general formula: 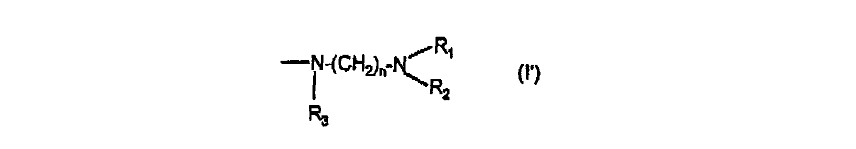 in which R₁ and R₂ are defined as above, R₃ represents a hydrogen atom or an alkyl radical and n is an integer from 2 to 4,
the alkyl portions or radicals defined above being straight or branched and containing 1 to 4 carbon atoms,
and its pharmaceutically acceptable salts, when they exist.

2. Cyclosporin derivative according to Claim 1, **characterized in that**:
- Alk represents an alkylene radical containing 2 to 6 carbon atoms in a straight or branched chain and
- R represents
• either a hydroxyl radical
• or an -NR₁R₂ radical in which R₁ and R_{z}, which are identical or different, represent hydrogen atoms or alkyl, alkenyl (2 to 4C) or optionally substituted (by a halogen atom, alkyloxy, alkyloxycarbonyl, amino, alkylamino or dialkylamino) phenyl radicals or represent benzyl radicals, or in which R₁ and R₂ form, with the nitrogen atom to which they are attached, a saturated or unsaturated heterocycle containing 4 to 6 ring members, which can contain another heteroatom chosen from nitrogen, oxygen or sulphur, optionally substituted by alkyl,
the alkyl portions or radicals defined above being straight or branched and containing 1 to 4 carbon atoms, and its pharmaceutically acceptable salts, when they exist.

3. Cyclosporin derivative according to Claim 1, **characterized in that**:
- Alk represents an alkylene radical containing 2 to 5 carbon atoms in a straight or branched chain and
- R represents
• either a hydroxyl radical
• or an -NR₁R₂ radical in which R₁ and R₂, which are identical or different, represent hydrogen atoms or alkyl, allyl, phenyl or benzyl radicals, or in which R₁ and R₂ form, with the nitrogen atom to which they are attached, a heterocycle chosen from azetidinyl, piperidyl, piperazinyl, N-methylpiperazinyl, N-phenylpiperazinyl, N-benzylpiperazinyl, morpholino, tetrahydropyridyl, methyltetrahydropyridyl or phenyltetrahydropyridyl,
the alkyl portions or radicals defined above being straight or branched and containing 1 to 4 carbon atoms, and its pharmaceutically acceptable salts, when they exist.

4. Cyclosporin derivative according to Claim 1, **characterized in that** it is [(R)-2-(N,N-dimethylamino)ethylthio-Sar]³-[4'-hydroxy-MeLeu]⁴-cyclosporin A, and its pharmaceutically acceptable salts.

5. Cyclosporin derivative according to Claim 1, **characterized in that** it is [(R)-2-(1-piperidyl)ethylthio-Sar]³-[4'-hydroxy-MeLeu]⁴-cyclosporin A, and its pharmaceutically acceptable salts.

6. Cyclosporin derivative according to Claim 1, **characterized in that** it is [(R)-2-(N-methyl-N-t-butylamino)ethylthio-Sar]³-[4'-hydroxy-MeLeu]⁴-cyclosporin A, and its pharmaceutically acceptable salts.

7. , Cyclosporin derivative according to Claim 1, **characterized in that** it is [(R)-2-(hydroxy)ethylthio-Sar]³-[4'-hydroxy-MeLeu]⁴-cyclosporin A, and its pharmaceutically acceptable salts.

8. Cyclosporin derivative according to Claim 1, **characterized in that** it is [(R)-2-(N,N-diethylamino)ethylthio-Sar]³-[4'-hydroxy-MeLeu]⁴-cyclosporin A, and its pharmaceutically acceptable salts.

9. Process for the preparation of a cyclosporin derivative according to Claim 1, **characterized in that** a disulphide of general formula:
R-Alk-S-S-Alk-R
in which R and Alk are defined as above, the functional groups of which which can interfere with the reaction have, if appropriate, been protected beforehand, is reacted with an activated form of a [4'-hydroxy-MeLeu]⁴-cyclosporin derivative of formula: 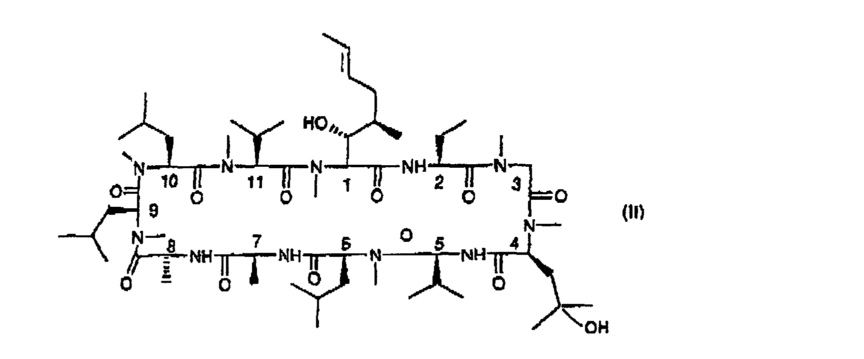 and then, if appropriate, the protective radicals are removed and/or the product obtained is optionally converted into a salt, when they exist.

10. Pharmaceutical composition, **characterized in that** it contains at least one product according to Claim 1, in the pure state or in combination with any compatible and pharmaceutically acceptable diluent or adjuvant and/or in combination with another antiviral, immunomodulating or antimicrobial active principle.

11. Synergizing combinations, **characterized in that** they comprise at least one cyclosporin derivative according to Claim 1 and at least one other agent known for its anti-retrovirus activity.

## Patentansprüche

1. Cyclosporin-Derivat, **dadurch gekennzeichnet, daß** es der allgemeinen Formel (I) 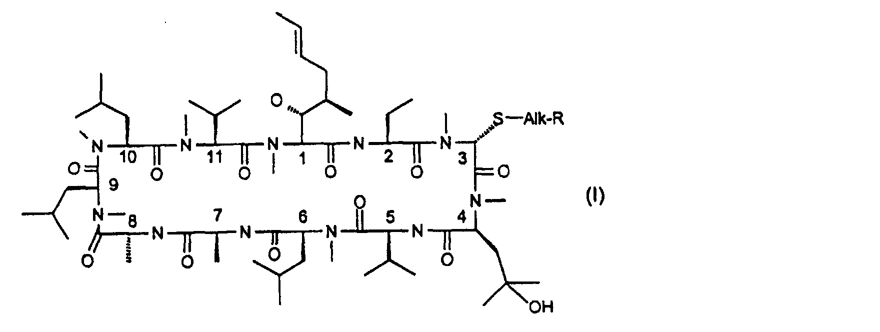 entspricht, in der:
- Alk einen Rest Alkylen mit 2 bis 6 Kohlenstoffatomen in gerader oder verzweigter Kette oder Cycloalkylen mit 3 bis 6 Kohlenstoffatomen darstellt, und
- R bedeutet:
. entweder einen Rest Hydroxy, Carboxy oder Alkyloxycarbonyl,
. oder einen Rest NR₁R₂, worin R₁ und R₂, gleich oder verschieden, Wasserstoffatome oder Reste Alkyl, Alkenyl (2 bis 4 Kohlenstoffatome), Cycloalkyl (3 bis 6 Kohlenstoffatome), Phenyl, gegebenenfalls substituiert (durch ein Halogenatom, Alkyloxy, Alkyloxycarbonyl, Amino, Alkylamino oder Dialkylamino), darstellen, oder gesättigte oder ungesättigte Reste Benzyl oder Heterocyclyl mit 5 oder 6 Ringgliedern und 1 bis 3 Heteroatomen bedeuten, oder worin R₁ und R₂ zusammen mit dem Stickstoffatom, an das sie gebunden sind, einen gesättigten oder ungesättigten Heterocyclus mit 4 bis 6 Ringgliedern bilden, der ein anderes Heteroatom enthalten kann, ausgewählt unter Stickstoff, Sauerstoff und Schwefel und gegebenenfalls substituiert durch Alkyl, Phenyl oder Benzyl,
. oder einen Rest der allgemeinen Formel (I') 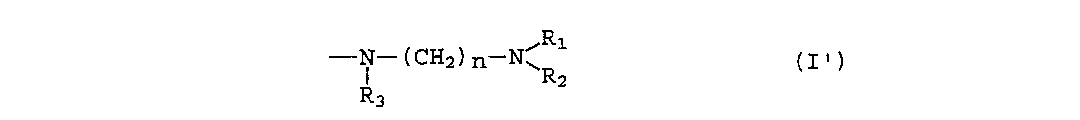 worin R₁ und R₂ wie oben definiert sind, R₃ ein Wasserstoffatom oder einen Rest Alkyl darstellt und n eine ganze Zahl von 2 bis 4 ist,
wobei die wie oben definierten Teile oder Reste Alkyl gerade oder verzweigt sind und 1 bis 4 Kohlenstoffatome enthalten,
sowie seine pharmazeutisch akzeptablen Salze, wenn diese existieren.

2. Cyclosporin-Derivat nach Anspruch 1, **dadurch gekennzeichnet, daß**
- Alk einen Rest Alkylen mit 2 bis 6 Kohlenstoffatomen in gerader oder verzweigter Kette darstellt, und
- R bedeutet:
. entweder einen Rest Hydroxy,
. oder einen Rest NR₁R₂, worin R₁ und R₂, gleich oder verschieden, Wasserstoffatome oder Reste Alkyl, Alkenyl (2 bis 4 Kohlenstoffatome), Phenyl, gegebenenfalls substituiert (durch ein Halogenatom, Alkyloxy, Alkyloxycarbonyl, Amino, Alkylamino oder Dialkylamino), darstellen, oder Reste Benzyl bedeuten, oder worin R₁ und R₂ zusammen mit dem Stickstoffatom, an das sie gebunden sind, einen gesättigten oder ungesättigten Heterocyclus mit 4 bis 6 Ringgliedern bilden, der ein anderes Heteroatom enthalten kann, ausgewählt unter Stickstoff, Sauerstoff und Schwefel und gegebenenfalls substituiert durch Alkyl,
wobei die wie oben definierten Teile oder Reste Alkyl gerade oder verzweigt sind und 1 bis 4 Kohlenstoffatome enthalten,
sowie seine pharmazeutisch akzeptablen Salze, wenn diese existieren.

3. Cyclosporin-Derivat nach Anspruch 1, **dadurch gekennzeichnet, daß**
- Alk einen Rest Alkylen mit 2 bis 5 Kohlenstoffatomen in gerader oder verzweigter Kette darstellt, und
- R bedeutet:
. entweder einen Rest Hydroxy,
. oder einen Rest NR₁R₂, worin R₁ und R₂, gleich oder verschieden, Wasserstoffatome oder Reste Alkyl, Allyl, Phenyl oder Benzyl darstellen, oder worin R₁ und R₂ zusammen mit dem Stickstoffatom, an das sie gebunden sind, einen Heterocyclus bilden, ausgewählt unter Azetidinyl, Piperidyl, Piperazinyl, N-Methylpiperazinyl, N-Phenyl-piperazinyl, N-Benzyl-piperazinyl, Morpholino, Tetrahydropyridyl, Methyl-tetrahydropyridyl, Phenyl-tetrahydropyridyl,
wobei die wie oben definierten Teile oder Reste Alkyl gerade oder verzweigt sind und 1 bis 4 Kohlenstoffatome enthalten,
sowie seine pharmazeutisch akzeptablen Salze, wenn diese existieren.

4. Cyclosporin-Derivat nach Anspruch 1, **dadurch gekennzeichnet, daß** es sich um [(R)-2-(N,N-Dimethylamino)-ethylthio-Sar]³-[4'hydroxy-MeLeu]⁴-cyclosporin A sowie seine pharmazeutisch akzeptablen Salze handelt.

5. Cyclosporin-Derivat nach Anspruch 1, **dadurch gekennzeichnet, daß** es sich um [(R)-2-(1-Piperidyl)-ethylthio-Sar]³-[4'-hydroxy-MeLeu]⁴-cyclosporin A sowie seine pharmazeutisch akzeptablen Salze handelt.

6. Cyclosporin-Derivat nach Anspruch 1, **dadurch gekennzeichnet, daß** es sich um [(R)-2-(N-Methyl-N-tert.-butylamino)-ethylthio-Sar]³-[4'-hydroxy-MeLeu]⁴-cyclosporin A sowie seine pharmazeutisch akzeptablen Salze handelt.

7. Cyclosporin-Derivat nach Anspruch 1, **dadurch gekennzeichnet, daß** es sich um [(R)-2-(Hydroxy)-ethylthio-Sar]³-[4'-hydroxy-MeLeu]⁴-cyclosporin A sowie seine pharmazeutisch akzeptablen Salze handelt.

8. Cyclosporin-Derivat nach Anspruch 1, **dadurch gekennzeichnet, daß** es sich um [(R)-2-(N,N-Diethylamino) -ethylthio-Sar]³-[4'hydroxy-MeLeu]⁴-cyclosporin A sowie seine pharmazeutisch akzeptablen Salze handelt.

9. Verfahren zur Herstellung eines Cyclosporin-Derivates nach Anspruch 1, **dadurch gekennzeichnet, daß** man ein Disulfid der allgemeinen Formel
R-Alk-S-S-Alk-R
worin R und Alk wie oben definiert sind und bei dem gegebenenfalls die Funktionen geschützt wurden, die in die Reaktion eingreifen können, auf eine aktivierte Form eines [4'-Hydroxy-MeLeu]⁴-cyclosporin-Derivates der Formel (II) 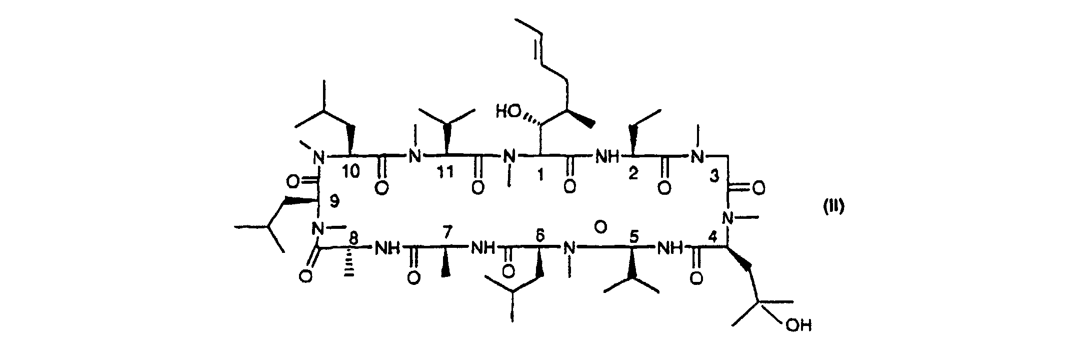 einwirken läßt,
anschließend gegebenenfalls die Schutzgruppen entfernt und/oder das erhaltene Produkt in ein Salz überführt, wenn diese Salze existieren.

10. Pharmazeutische Zusammensetzung, **dadurch gekennzeichnet, daß** sie mindestens ein Produkt nach Anspruch 1 in reinem Zustand oder in Assoziation mit jedem kompatiblen und pharmazeutisch akzeptablen Verdünnungsmittel oder Zusatzstoff und/oder in Assoziation mit einem anderen antiviralen, immunomodulierenden oder antimikrobiellen Wirkstoff enthält.

11. Synergistische Assoziationen, **dadurch gekennzeichnet, daß** sie mindestens ein Cyclosporin-Derivat nach Anspruch 1 und mindestens ein anderes Mittel umfassen, das für seine Anti-Retrovirus-Aktivität bekannt ist.
